# EUROPEAN PATENT SPECIFICATION

(11) **EP 0 612 308 B1**
(45) Date of publication and mention of the grant of the patent: **05.02.1997**
(21) Application number: 92923264.3
(22) Date of filing: 06.11.1992
(51) Int. Cl.: C07B 59/00, C07C 229/36, C07C 233/47

(54) **METHOD FOR PREPARING RADIO-IODINATED THYRONINE DERIVATIVES; NOVEL INTERMEDIATES**
VERFAHREN ZUR HERSTELLUNG VON RADIOJODTHYRONIN-DERIVATEN; NEUE ZWISCHENPRODUKTE
PROCEDE DE PREPARATION CONCERNANT DES DERIVES DE THYRONINE CONTENANT DE L'IODE RADIOACTIF, NOUVEAUX PRODUITS INTERMEDIAIRES

(30) Priority: 08.11.1991 DE 4136850
(43) Date of publication of application: 31.08.1994
(73) Proprietor: HENNING BERLIN GMBH, D-12099 Berlin (DE)
(72) Inventor: THOMA, Rudy, D-1000 Berlin 42 (DE)
(74) Representative: Andrae, Steffen, Dr.
(86) International application number: EP9202554
(87) International publication number: WO9309076

(56) References cited:
- US-A- 4 192 858
- JOURNAL OF THE CHEMICAL SOCIETY, 1961, LETCHWORTH GB pages 2890 - 2902 A. DIBBO ET AL 'The Synthesis of Thyroxine and Related Compounds. Part XVII. The Preparation of Some Additional Compounds related to Thyroxine'
- JOURNAL OF THE CHEMICAL SOCIETY, PERKIN TRANSACTIONS 1. 1988, LETCHWORTH GB pages 3103 - 3111 D. M. B. HICKEY ET AL 'Synthesis of Thyroid Hormone Analogues. Part 3. Iodonium Salt Approaches to SK &F L-94901'
- CHEMICAL ABSTRACTS, vol. 104, no. 25, 23 June 1986, Columbus, Ohio, US; abstract no. 225191d, P. HRADILEK ET AL 'LABELING OF TYROSINES AND THYRONINES WITH IODINE RADIOISOTOPES' page 645 ;column 1 ;
- JOURNAL OF NUCLEAR MEDICINE. vol. 15, no. 10, October 1974, NEW YORK US pages 863 - 867 R. M. LAMBRECHT ET AL 'A NOVEL 123-I-LABELING REAGENT. XIII. SYNTHESIS AND LOADING-DOSE EFFECTS OF 123-I-4-IODOPHENYLALANINE AND 123-I-5- AND 6-IODOTRYPTOPHAN'
- CHEMICAL REVIEWS vol. 82, no. 6, 1982, EASTON US pages 575 - 590 R. H. SEEVERS ET AL 'RADIOIODINATION TECHNIQUES FOR SMALL ORGANIC MOLECULES'
- JOURNAL OF ORGANIC CHEMISTRY. vol. 47, no. 8, 9 April 1982, EASTON US pages 1484 - 1488 T. J. MANGNER ET AL 'SOLID-PHASE EXCHANGE RADIOIODINATION OF ARYL IODIDES. FACILITATION BY AMMONIUM SULFATE'

## Description

The invention relates to novel methods for introducing one or two radioactive iodine isotopes into the 3-position or 3,5 positions of the α-ring of an iodine thyronine compound and novel compounds which are particularly suitable as intermediates for these methods. The method according to the present invention is also suitable for preparing the corresponding thyronamine and thyrocarboxylic acid derivatives. If in the following description reference is made to "iodine thyronine compounds" or "halogen thyronines" or "thyronine compounds" in connection with the present invention, the corresponding statements shall also apply to thyronamine and thyrocarboxylic acid derivatives, unless a skilled person would understand that the corresponding specific passage would not permit such a broad interpretation of the above-mentioned terms.

The introduction of radioactive iodine isotopes, in particular the introduction of ¹²⁵I, into the β-ring of iodo thyronine compounds such as triiodo thyronine (T3) or tetraiodo thyronine (T4) does not pose any difficulties in practical terms and can be regarded as a routine measure. By contrast, hardly any methods are available which would allow one or two iodine isotopes to be selectively introduced as labels into the α-ring of iodo thyronine compounds. However, iodo thyronine compounds labelled with radioactive iodine isotopes in the α-ring are of scientific and practical interest for various reasons.

On the one hand, such compounds are needed when 3,5-diiodo thyronine (T2) is to be determined by classical immuno diagnostic methods such as RIA. On the other hand, thyronine compounds labelled with radioactive iodine isotopes in the α-ring have been found to possess a greater chemical stability compared to the corresponding compounds labelled in the β-ring which have been used so far. This greater chemical stability can be of advantage when the compounds are used as tracers in known immunoassays, as it allows an improvement in the storability and sensitivity and a reduction of the interfering background to be achieved.

The thyroid hormones triiodo thyronine (T3) and tetraiodo thyronine (T4) radioactively labelled in the α-ring are of importance in research in connection with the investigation of metabolic processes, in particular in studies of the deiodinase type III.

It is true that methods which may lead to iodo thyronine compounds with a radioactive iodine labelling in the α-ring have been described in the literature, viz. in J. Org. Chem. 27 (1962), pages 1773 to 1778 and in Endocrinology 1977, vol. 101, No. 4, pages 1276 to 1280. However, these methods suffer from the disadvantage involved in the procedure of first preparing a radioactively labelled tyrosine and then subjected it to a chemical reaction with suitable reactants to yield thyronine derivatives labelled in the α-ring. This procedure has the disadvantage of involving chemical multistep reactions with radioactively labelled compounds which is highly disadvantageous both from a safety point of view and from the viewpoint of the scale on which the chemical reactions can be carried out which is determined by the amounts of labelled starting compounds available.

It would be advantageous to possess a method which permits the introduction of the radioactive iodine isotopes into the α-ring of the otherwise largely finished iodo thyronine compound. As the α-ring in the thyronine compounds does not have the activating phenolic OH group of the β-ring, it was actually obvious to perform the halogen exchange by electrophilic radio-iodination using the method described in J. Org. Chem. 1982, 47, pages 1484 to 1488 which had been developed for the radio iodination of non-activated aryl iodides, wherein the iodine exchange in the non-activated aryl iodides occurs in the presence of ammonium sulfate for instance with Na¹²⁵I under oxidizing conditions. Although, according to the literature, this method proved to be successful with compounds such as (m-iodo benzyl) guanidine even when the melting point was not achieved, this method failed completely when the attempt was made to use it for the radio-iodination of the α-ring of the iodo thyronines.

In iodo thyronines, the above-mentioned method did not produce an exchange even at 200°C (see the following comparative example).

Radioisotopy 25 (5), 675-684 (1984) (Czech.) (cf. also C.A. 104: 225191d) describes the labelling of iodinated tyrosines and thyronines with iodine radioisotope by the cautious iodination of the corresponding non-iodinated derivative with elementary radioiodine.

In: The Journal of The Chemical Society, 1961, pages 2890-2902 among other compounds the preparation of the ethyl ester of N-acetyl-3,5-dibromo-4-p-methoxyphenoxy-phenylalanine is disclosed. Said compound is further demethylated with hydroiodic acid to give 3,5-dibromothyronine which then is further halogenated in the β-ring in order to obtain higher halogenated thyronine derivatives.

In: Journal of The Chemical Society Perkin Transactions I, 1988, pgaes 3103 to 3111 among other compounds the preparation of N-trifluoroacetyl-3,5-dibromo-4-p-methoxyphenoxy-phenylalanine as undesirable by-product of the preparation of L-3,5-Dibromo-3'-[(6-chloropyridazin-3-yl)methyl]-4'-O-methyl-N-trifluoroacetylthyronine methyl ester is disclosed.

In: The Journal of Nuclear Medicine, Vol. 15, No. 10, pages 863 to 867 (1974) the radioiodination of ¹²³I-4-iodophenylalanine and ¹²³I-5- and 6-iodotryptophan by a melt method is described. The starting compounds do not contain ether bonds and an activated β-ring as it is present in thyronine compounds.

The present invention addresses the problem of providing a method whereby the α-ring of iodo thyronine compounds can be radio-iodinated in a simple and effective manner, yielding radio iodinated compounds which preferably show a high specific activity and are preferably free from any carrier substances.

This problem is solved by a method according to patent claims 1 and 2, respectively.

Advantageous embodiments of the method according to the invention are specified in the subclaims.

A particularly advantageous variant of the method according to the invention starts from intermediates in the form of a low melting derivative of a 3-bromo or 3,5-dibromo thyronine compound according to formula Ia, which to applicant's knowledge have not yet been described in the literature. They are therefore claimed as valuable intermediates.

The present invention is based on the surprising finding that it is possible to radio iodinate halogen thyronine compounds in the α-ring using an iodine nuclide, after previously subjecting the corresponding compound to a treatment geared at the formation of a relatively low melting derivative which can then be radio iodinated in the molten state with an iodine nuclide, resulting in a halogen or inter-halogen exchange. The substituents introduced for the formation of derivatives can then be removed in the same reaction vessel, yielding the desired iodo thyronine which are radio iodinated in the α-ring.

Moreover, the method according to the invention also has the advantage that, when using 3-bromo thyronine compounds or 3,5-dibromo thyronine compounds as starting materials, the radio iodination products are easily separated by chromatography from the compounds used as starting material and the compounds resulting from it after the residues R¹, R^{2,} R³ have been split off. The reason for the afore-mentioned easy separation, which allows the iodo thyronine compounds radio iodinated in the α-ring to be obtained free from carrier substances, that is to say free from non-radio iodinated compounds, is the fact that the organic molecules formed in the radio iodination have different substituents.

If the radio iodination according to the invention is carried out using thyronine compounds which are unsubstituted in the β-ring, for instance T2-derivatives, then the thyronine compounds radio iodinated in the α-ring and obtainable in pure form can subsequently be substituted in the β-ring, which is easier to substitute, and can be iodinated or radio iodinated in particular in the 3' or 3',5' positions. This way, triiodo thyronine or tetraiodo thyronine derivatives being radio iodinated both in the α-ring and possibly in the β-ring too can be obtained. In this case, it is also possible to introduce different radio iodine isotopes into the α-ring and β-ring, which may be advantageous for particular objectives, for instance in investigations of the metabolism. Moreover, the method according to the invention can also be used to obtain iodo thyronine compounds which are free from carrier substances and possess a very high specific activity.

The method according to the invention, including the preparation of the required starting compounds is hereinafter explained in more detail and the preferred embodiments are further documented by working examples.

In its most general form, the present invention relates to a method for preparing iodo thyronine compounds that are labelled in the α-ring and can be characterized by the following general formula (II): 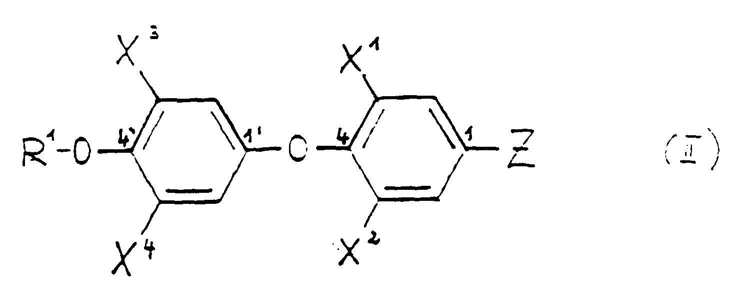 in which
R¹ is H or an optionally substituted linear or branched alkyl or cycloalkyl group with 1 to 8 carbon atoms, or phenyl, or aralkyl with 7 to 14 carbon atoms,
X¹ is one of the radioactive iodine isotopes ¹²³I, ¹²⁵I or ¹³¹I,
X² is likewise one of the radioactive iodine isotopes mentioned in connection with X¹, or H, Br, ¹²⁷I, Cl, SCN, CN or a linear, branched or cyclic alkyl having 1 to 7 carbon atoms.
X³ and X⁴ are independently from each other H, ¹²⁷I, ¹²³I, ¹²⁵I or ¹³¹I or another substituent introduced subsequently, such as Br, I, F, SCN, CN, NO₂, NH₂, OH or alkyl, aralkyl or aryl groups with 1 to 7 carbon atoms,
Z is one of the groups -(CH₂)ₙ-COOR², -CH₂-CH₂-NHR³ or -CH₂-CH(NHR³)-COOR², wherein
R² is defined as R¹ and stands for the same group as R¹ or a group different therefrom,
R³ is H or an acyl or a linear, branched or cyclic alkyl with 1 to 7 carbon atoms each, and
n can be 0, 1 or 2.

Consequently, the method according to the invention can also be used to obtain the thyronamine derivatives of the following general formula (IV) and the thyro carboxylic acid derivatives of the general formula (V), in which all groups are defined as in the general formula (II):

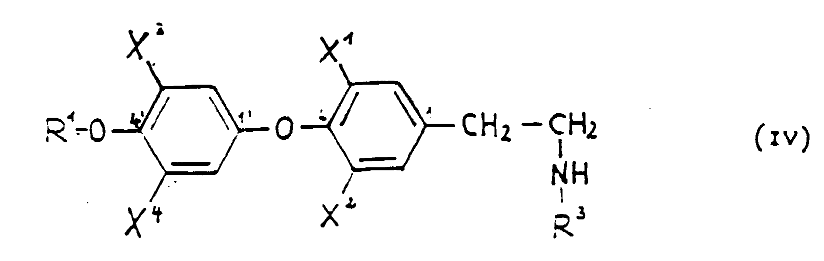

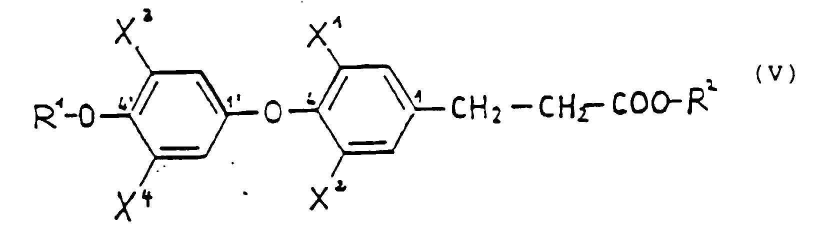

By subjecting derivatives with a correspondingly low melting point to radio iodination according to the invention, it is possible to obtain, instead of the thyrocarboxylic acid derivatives (V) (n = 2), compounds in which the propionic acid group is substituted in the 1-position for instance with a carboxylic group or with an acetyl group (n = 0 or 1). The necessary starting materials are described for instance in J. Org. Chem. (1957), 22, pages 1577-1581 or J. Chem. Soc. (1951), p. 2467, which when subjected to esterification or phenol ether formation can yield compounds that can be successfully used in the method according to the invention. The preparation of 3,5-diiodo thyronamine is described in Bull. Soc. Chim. France, 4, 716 (1962). In addition, 3,5-diiodo thyro acetic acid (Diac) and 3,5-diiodo thyronamine (Diam) are obtainable from the Applicant as commercial products. 3,5-diiodine thyro propionic acid can also be obtained from Sigma (Sigma D 7032).

In the method according to the invention, the optionally present iodine or radio iodine substituent X³ and the optionally present X⁴ (just as the other substituents mentioned as alternatives) are introduced in a later reaction step, preferably by iodination with sodium iodide/chloramine T at the β-ring, as for instance described in Nature, vol. 194, 495 to 496 (1962) or in Scand. J. Clin. Lab. Invest. 32, 357 to 360 (1973). The iodine atoms introduced at this stage may be normal iodine atoms of the stable isotope ¹²⁷I, but may also be radio isotopes of the type mentioned above. Moreover, any other substitution reaction is also possible at this stage, should such a reaction be desired for any reason.

The first final stage of the method according to the invention is thus achieved by obtaining compounds containing one or two iodine nuclides in the α-ring, while X³ and X⁴ continue to stand for H in the β-ring. Consequently, these compounds are also valuable intermediates, which can be used to obtain compounds carrying almost any substituent in the 3',5'-positions of the β-ring (for instance Cl, Br, SCN, CN, NO₂, NH₂, OH, alkyls aryls or aralkyls), employing conventional methods and simple reactions.

While X¹ always stands for a radioactive iodine isotope, the meaning of X² depends on the starting compound used in the method and possibly the reaction conditions (reaction time, reaction temperature) and the relative molar amounts of the alkali metal radio iodide used in the method. If the starting compound is a compound in which X² stands for H from the beginning, the meaning of the group X² depends on the type of the second substituent that is originally present and possibly also on the reaction conditions chosen, as will be explained in the following once again in more detail.

The method according to the invention starts from compounds without radioactive labels which can be defined by the general formula I: 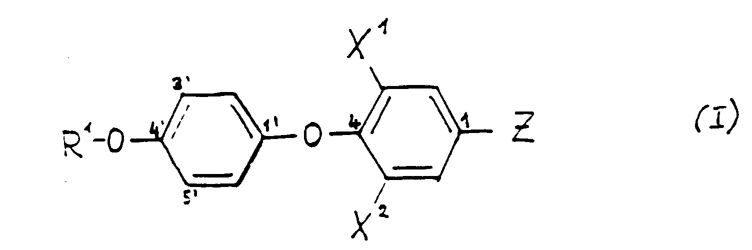 in which
- R¹: is an optionally substituted linear or branched alkyl or cycloalkyl having 1 to 8 carbon atoms, phenyl, or an aralkyl having 7 to 14 carbon atoms,
- X¹ and X²: may be the same or different and stand for Br, I, H, Cl, SCN, CN or i-C₃H₇, with the proviso, that at least one of these residues is a group exchangeable for a radioactive iodine isotope,
- Z: is one of the groups -(CH₂)ₙ-COOR², -CH₂-CH₂-NHR³ or -CH₂ -CH(NHR³)-COOR², in which
- R²: is defined as R¹ and stands for the same group as R¹ or a group different from it,
- R³: stands for H or an acyl having 1 to 7 carbon atoms or a linear, branched or cyclic alkyl having 1 to 7 carbon atoms, and
- n: may be 0, 1 or 2.

For preparing proper iodo thyronine compounds, compounds of the general formula (Ia) (Z = -CH₂-CH(NHR³)-COOR²) are used as starting compounds. 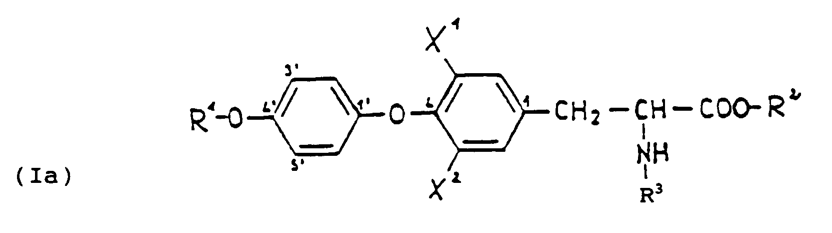 in which the residues R¹, R², R³ X¹ and X² are defined as in the general formula (I).

For preparing thyronamine compounds or carboxylic acid compounds, corresponding compounds are used in which Z has one of the other two meanings of formula (I) (Z = -CH₂-CH₂-NHR³ or -(CH₂)ₙ-COOR²).

For the reasons explained in the following in more detail, the compounds of the above-mentioned general formula are particularly advantageous, in which X¹ or both X¹ and X² stand for Br.

The compounds of formula Ia can be prepared as follows:

The starting material used is a tyrosine of the formula (III) 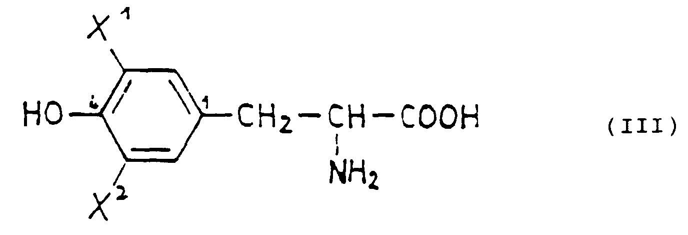 in which X¹ and X² have the meaning as specified in formula I. Such tyrosines are known compounds, some of which are commercial products. 3,5-diiodo tyrosine and the 3,5-dibromo tyrosine, for instance, are compounds which can be purchased from the company Sigma under the designation Sigma D0754 and D5632, respectively. 3-iodo tyrosine can be purchased from the company Sigma under the designation Sigma I8250.

3-bromo tyrosine, which leads to the important intermediate 3-bromo thyronine, is not a commercial product. It can for instance be prepared according to the method described in Example 6 below.

In order to form the thyronine structure, the compounds are reacted with an O-arylating agent, which links the residue 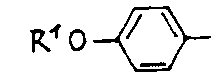 to the phenolic HO group of the starting tyrosine compound III while undergoing esterification. The diaryl iodonium compounds, such as the di(p-anisyl) iodonium bromide, is a suitable O-arylating agent.

The reaction product is a compound of the general formula Ia, in which R² and R³ are both H.

To obtain a derivative with a melting point as low as possible, namely below 180°, preferably below 145°, and in general as low as possible, the group R² is introduced into the molecule by a known esterification reaction, as is optionally the group R³, by a subsequent N-acylation or N-alkylation reaction, R² and R³ having the meanings as defined in formula I above. Moreover an N-acetylation (R³=COCH₃) in situ can occur, if the compound with the free NH₂ group (R³ = H) is subjected to the below-described nuclide exchange in the presence of glacial acetic acid.

Surprisingly, these compounds have been found to permit a direct halogen exchange or even interhalogen exchange for a radioactive iodine isotope in the 3- and optionally 5-positions, if they are reacted with Na¹²³I, Na¹²⁵I or Na¹³¹I at temperatures in the vicinity of their melting point under suitable reaction conditions. Depending on the type of the substituents X¹ and X² of the general formula I and the molar amount of the radio iodide used, one or both of the afore-mentioned residues are exchanged for the radioactive iodine isotope provided as alkali iodide or alkaline earth iodide. Provided that the alkali or alkaline earth radio iodide is used in a sufficient molar excess amount, it is possible to obtain a di-radio-iodinated product, if the starting compounds used have two exchangeable residues. The usual molar amounts for a single iodine exchange are preferably employed to obtain a mono-radio-iodinated product. If a dibromo compound is used as the starting material, the reaction can be stopped even more selectively after the introduction of a radioactive iodine atom, as in this case the interhalogen exchange is slower. The introduction of only one radio iodine atom can also be promoted by using the starting compound in excess amounts. As the unreacted dibromo compound can be easily separated from the mono-iodinated reaction product, for instance by a chromatographic process, such as reverse phase gradient chromatography, the radio iodinated compound can be obtained by this method in a form free from carrier substances, that is to say in a form free from unlabelled material.

Although the reaction can, on principle, be stopped after the nuclide exchange occurred, yielding compounds which still contain the substituents R¹, R² and R³, and the reaction mixture can be worked up, the reaction is usually continued in such a way that the substituents R¹, R² and R³, which are supplied to the same reaction vessel or reaction batch in order to lower the melting point, are split off according to known methods, for instance by hydrolysis.

For this purpose, an acid suitable for producing the desired splitting off of the ether or ester or N-acyl groups is added to the reaction mixture, which is then heated in the closed reaction vessel for several hours to temperatures above 100°C.

The foregoing statements show that the compounds of the general formula I in which both X¹ and X² stand for Br, offer special advantages for the separation of the radio-iodinated compounds formed in the interhalogen exchange of bromine/iodine. These advantages are obtained even if the interhalogen exchange occurs in a manner different from that described above. Similar advantages are obtained when using the corresponding mono-bromated starting compound (X¹=Br, X²=H).

Therefore, the afore-mentioned bromo compounds are valuable intermediates, and as these compounds have not yet been found to have been known in the chemical literature, they are claimed in the present invention as valuable key intermediates for the preparation of iodine thyronine compounds labelled in the α-ring.

In the following, the invention is explained in more detail by working examples, which are not to be construed as a limitation of the claims, but are to serve the better understanding of the terms used in the preceding description.

### Example 1

### Preparation of the starting compounds for the method according to the invention - preparation of N-acetyl-3,5-dibromo-(4'-O-methyl)-thyronine methylester

### a) Preparation of 3,5-dibromo-(4'-O-methyl)-thyronine:

0.02 mole (6.78 g) of 3,5-dibromo-L-tyrosine (based on Sigma D 5632) and 0.014 mole copper(II)-hydroxide were suspended in 200 ml of water under a nitrogen atmosphere. This mixture was mixed with 0.026 mole of di(p-anisyl)iodonium bromide and 0,026 mole of triethylamine and heated to 60°C. After 45 minutes, additional di(p-anisyl)-iodoniumbromide was added in an amount of 0.13 mole and the mixture was kept at 60°C for another 45 minutes. The hot mixture was then mixed with 5 ml of concentrated hydrochloric acid and cooled in an ice bath while being stirred. The precipitate was removed by filtration, washed with water until its pH turned neutral and suspended four times in 100 ml of toluene each and filtered. The resulting washed precipitate was dissolved in 100 ml of a mixture of methanol and concentrated hydrochloric acid (10:1) and treated with 0.5 g of activated charcoal. The solution was then poured into approximately 3 l of water while being stirred. The precipitate which formed was removed by filtration, washed with water until its pH turned neutral and dried in the desiccator.

Yield: 2.64 g (0.006 mole) = 29.8% based on the tyrosine used.

### b) 3,5-dibromo-(4'-O-methyl)-thyronine methyl ester

2 g of the 3,5-dibromo-(4'-O-methyl)-thyronine obtained in Example la) and 2 g of p-toluene sulfonic acid were dissolved in 70 ml of methanol and 200 ml of toluene and refluxed in a Soxhlet apparatus for 8 hours, the jacket of which contained a desiccant. In order to work the mixture up, the solvents were removed from the reaction solution and the resultant dry residue was dissolved in a small amount of ethanol, the solution was treated with activated charcoal and the product was precipitated from the ethanolic solution by the addition of water. After filtering, the reaction product was dried in the vacuum desiccator.

The yield of 3,5-dibromo-(4'-O-methyl)-thyronine methyl ester was 1.5 g (74%), the melting point of the product obtained in the above-described manner, without being subjected to a further purification process was 47.5 to 53°C.

### c) Preparation of N-acetyl-3,5-dibromo-(4'-O-methyl)-thyronine methylester

For the N-acetylation of the product of Example 1b), 1 g (2.15 mmoles) of 3, 5-dibromo-(4'-O-methyl)-thyronine methylester from the preceding step 1b) was dissolved in 10 ml of tetrahydrofuran and allowed to stand together with 0.44 g (4.3 mmoles) of acetic acid anhydride and 2 ml of triethylamine at room temperature for 3 hours. To work the solution up, the solution was evaporated to dryness and the residue dissolved in some ethanol. The acetylated reaction product was precipitated from the ethanolic solution by dilution with water. The precipitate was removed by filtration and dried in the vacuum desiccator.

The N-acetyl-3,5-dibromo-(4'-O-methyl)-thyronine methyl ester yield was 0.9 g (82.5%). The melting point was found to be 115°C.

Within the usual error margin, the elementary analysis corresponded to the values calculated.

### Example 2

### Preparation of the N-acetyl-3,5-diiodo-(4'-O-methyl)-thryronine ethylester

Example 1 was repeated using 3,5-diiodo tyrosine (Sigma D0754) of example la) as the starting material and performing the esterification in step 1b) with ethanol instead of methanol.

The compound 3,5-diiodo-(4'-O-methyl)-thyronine ethylester had a melting point of 77.5 to 80°C, and the N-acetylation product prepared from it had a melting point in the range of 126 to 141°C.

Within the usual error margin, the elementary analysis corresponded to the values calculated.

### Example 3

### Introduction of ¹²⁵I into the compound N-acetyl-3,5-diiodo-(4'-O-methyl)-thyronine ethyl ester by nuclide exchange

10 µg of N-acetyl-3,5-diiodo-(4'-O-methyl)-thyronine ethyl ester (prepared according to Example 2) in the form of an alcoholic solution were placed with a pipette onto the bottom of a conical reaction vessel (Aldrich Z11, 514-2), permitting a high mass concentration in a small volume, and the alcohol was evaporated in a slight nitrogen flow in such a way that a splashing of the solution in the vessel was prevented. The dry residue in the reaction vessel was then mixed with 1mCi of a ¹²⁵I-sodium iodide solution (Amersham IMS 30) and 10 µl of glacial acetic acid. The reaction vessel was closed and heated for 2 hours at 120° to 130°C.

Right after cooling, the resulting reaction product was mixed with 100 µl of glacial acetic acid and 100 µl of concentrated hydrochloric acid. The reaction vessel was closed and heated for a further 5 hours at 120° to 130°C.

After cooling, the batch was diluted with 1 to 2 ml of water and purified over a reverse-phase gradient chromatography, to work it up.

The HPLC system used comprises the following:

A stationary phase: Eurospher 80 C18 5 µ (Company Eurochrome, Berlin)
- a mobile phase:: A = 10% of acetonitrile/0.1% of trifluoro acetic acid
B = 90% of acetonitrile/0.1% of trifluoro acetic acid
- a gradient:: from 0% B to 100% B in 30 minutes;
- flow rate:: 1.5 ml/min.
- 1. detector:: UV 210 nm;
- 2. detector:: radio activity.

The peak corresponding to 3,5-diiodine thyronine was collected and optionally rebuffered depending on the envisaged later use.

Before the final preparative purification, the labelling yield was determined in an aliquot of the solution, for instance by integrating the signals from the radio activity detector of an analytic HPLC system. The method described, resulted in a labelling of more than 95% and led to specific activities of 100 mCi/mg.

### Example 4

### Introduction of ¹²⁵I into the compound N-acetyl-3,5-dibromo-(4'-O-methyl)-thyronine methyl ester by nuclide exchange

Example 3 was repeated using as a starting compound the reaction product obtained in Example 1c).

As in this case the product of the nuclide exchange and/or the end product 5-¹²⁵I,3-bromo-thyronine obtained after hydrolysis differed chemically from the starting material 3,5-dibromo thyronine, it was possible to use the starting compound in an excess amount, as the non-radio iodinated 3,5-dibromo thyronine was easy to separate in a subsequent chromatographic purification from the products formed by the introduction of ¹²⁵I.

In this way, it was possible to obtain the resulting product 5-¹²⁵I, 3-bromo-thyronine free from carrier substances and without impairment of its specific activity.

### Example 5

The radio iodinated compounds resulting after the nuclide exchange according to Examples 3 and 4 can be subjected to a further iodination of the unsubstituted β-ring in a known manner either with radioactive or non-radioactive iodine. This way it is possible to produce a whole spectrum of iodine thyronine compounds labelled in the α-ring. If in the iodination in the β-ring, which can be carried out in the usual manner with sodium iodide/chloramine T, a radioactive iodine isotope is introduced, it is possible to obtain T3 and T4 compounds with a particularly high radioactive labelling. The iodine isotope used for the β-iodination may be different from the one used for the labelling of the α-ring.

This way, the following can, for instance, be obtained:

When starting from the compound of formula I, in which X¹ = X² = I, and carrying out a nuclide exchange according to Examples 3 and 4, the following can be obtained: 3¹²⁵I,5-diiodo thyronine, which can be subjected to a further mono or diiodination, for instance with Na¹²⁷I in the β-ring to give 3',3¹²⁵I,5-triiodo thyronine or 3',5',3¹²⁵I, 5-tetraiodo thyronine.

In an analogous manner, 3-iodo thyronine can be subjected to a nuclide exchange to yield 3¹²⁵I-monoiodo thyronine, which can in turn be subjected to cold iodination of the β-ring to give 3',3¹²⁵I-diiodo thyronine and 3',5',3¹²⁵I-triiodo thyronine. Using 3-bromothyronine as a starting material, the afore-mentioned compounds are obtained free from carrier substances.

When 3,5-dibromo thyronine is used as the starting material and subjected to a mono nuclide exchange, it will yield 5-bromo-3¹²⁵I-monoiodo thyronine, which when subjected to cold iodination of the β-ring will produce the corresponding compounds 5-bromo-3',3¹²⁵I-diiodo thyronine and 5-bromo-3',5',3¹²⁵I-triiodo thyronine.

The compounds of the formula I, in which X² is one of the other residues mentioned, can be used as starting materials to give compounds which are analogous to the afore-mentioned bromine compounds and incorporate for instance a chlorine group, CN-group, SCN group or an I-C₃H₇ residue, instead of the 5-bromo residue.

### Comparative Example

### Attempt to carry out the iodine exchange in 3,5-diiodo thyronine

Amounts of up to 10 mg of 3,5-diiodo thyronine and 1mCi ¹²⁵I-sodium iodide, both in this mixture and with the addition of ammonium sulfate or glacial acetic acid or with the addition of ammonium sulfate and glacial acetic acid were heated in a conical reaction vessel at temperatures above 160°C for several hours. The corresponding reaction mixtures were then dissolved in water/methanol and examined by HPLC.

The HPLC system consisted of a reverse-phase gradient apparatus equipped with a UV and a radioactivity detector.

The test showed that none of the runs carried out according to this example resulted in the incorporation of radioactive material into the 3,5-diiodo thyronine. Nor was there any other peak, which would have shown UV and radioactivity signals at the same time.

The same result was obtained with a radio activity scanner in the thin layer chromatography test.

### Example 6

### Preparation of 3-bromotyrosine

3-bromotyrosine was prepared in a conventional manner by monobromating 3-(4-methoxyphenyl)-alanine (Aldrich 15,825-9) and subsequent hydrolysis. For this purpose, 10 g (0.051 mole) of 4-O-methyltyrosine were dissolved in 50 ml 98 percent formic acid and 8.15 g (0.051) of bromine were added dropwise by means of a pipette at 0°C-5°C for 1,5 hours while stirring. The reaction mixture was continued to be stirred at room temperature over night and was then diluted with 150 ml of dilute hydrochloric acid (10%), then briefly heated to a boil and subsequently evaporated to dryness in the vacuum rotation evaporator.

The solid residue was placed into 50 ml of hot water, optionally treated with activated charcoal, and crystallized by cooling and neutralizing the solution. After filtration and washing with water, the 3-bromo-4-O-methyl tyrosine obtained as the filtration residue was dried in the vacuum desiccator (see also J. Org. Chem., vol. 50, No. 24, 1985, pages 4909-4913 and the literature passages cited in it).

Yield: 10.5 g (76% of the theory). Boiling point: 223-226°C (degradation).

This compound was hydrolized in a known manner and heated for several hours with concentrated hydrochloric acid/glacial acetic acid or concentrated hydriodic acid/glacial acetic acid to give the free 3-bromotyrosine.

### Example 7

### Preparation of N-acetyl-3-bromo-(4'-O-methyl)-thyronine

This compound was prepared using 3-bromotyrosine as the starting compound and subjecting it to the same treatment as described in Example 1.

## Claims

1. A method for preparing iodo thyronine derivatives which are labelled in the 3-position or 3,5-positions of the α-ring with one or two radioactive iodine isotopes.
characterized by the following steps:
a) carrying out a halogen exchange in a 3-mono- or 3,5-di- substituted thyronine derivative of the general formula I 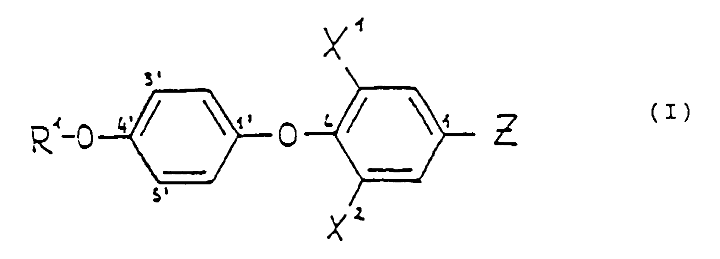 in which
R¹ is an optionally substituted linear or branched alkyl or cycloalkyl having 1 to 8 carbon atoms, a phenyl or an aralkyl having 7 to 14 carbon atoms,
X¹ and X² may be the same or different and stand for Br, I, H, Cl, SCN, CN or I-C₃H₇, with the proviso, that at least one of these residues is a group exchangeable for a radioactive iodine isotope,
Z is one of the groups -(CH₂)ₙ-COOR², -CH₂-CH₂-NHR³ or -CH₂-CH(NHR³)-COOR², in which
R² is defined as R¹ and stands for the same group as R¹ or a group different from it,
R³ stands for H or an acyl having 1 to 7 carbon atoms or a linear, branched or cyclic alkyl having 1 to 7 carbon atoms, and
n may be 0, 1 or 2,
in which the combination of the substituents R¹, R² and R³ is so chosen that the compound has a melting point below 180°C, by reacting a compound of formula I at temperatures in the vicinity of or above their melting point with a suitable alkali metal or alkaline earth metal iodide containing a radioactive iodine isotope, and optionally isolating the resulting compound or
b) splitting off the residues R¹ and/or R² and/or R³ from the reaction product of step a) and recovering the corresponding unsubstituted iodo thyronine compound labelled with a radio-active iodine isotope in the α-ring and isolating the resulting reaction products
and optionally additionally
c) introducing in a conventional manner one or two iodine atoms or another substituent from the group consisting of the residues Br, Cl, F, SCN, CN, NO₂, NH₂OH and alkyl groups, aralkyl groups and aryl groups containing up to 7 carbon atoms, into the 3' position or the 3',5' positions of the β-ring of the radio iodinated compounds obtained in steps a) or b), whereby higher substituted iodo thyronine compounds labelled with radioactive iodine at least in the α-ring are obtained.

2. The method according to claim 1, characterized in that in step a) a compound of the general formula I is used, in which Z stands for -CH₂-CH(NHR₃)-COOR² and in which R¹ and R² are independently from each other alkyl groups with 1 to 4 carbon atoms, R³ is a formyl or acetyl group and X¹ and X² are independently from each other Br and/or I-atoms, one of these residues possibly also being H, and in that the melting point of the compound is below 145°C.

3. The method according to claim 2, characterized by using in step a) a compound of the general formula I, in which R¹ is methyl, R² is methyl or ethyl, R³ is H or acetyl and X¹ and X² are both Br or I.

4. The method according to claim 1, characterized in that the alkali metal iodide used in step a) is Na¹²³I, Na¹²⁵I or Na¹³¹I.

5. The method according to claim 1, characterized in that step a) is carried out at temperatures in the range from 120° to 140°C.

6. The method according to claim 1, characterized in that the reaction in step a) and optionally step b) is carried out in the tip portion of a conical reaction vessel, in an ampoule or in a tube for determining the melting point.

7. The method according to one of the claims 1 to 6, characterized in that the steps a) and b) are carried out in the same reaction vessel without intermediate isolation of the product of step a), wherein for splitting off the residues R¹, R² and optionally R³ an acid is added to the reaction product obtained in step a) and the reaction is continued while heating is continued.

8. The method according to claim 7, characterized in that the mono- or di- radio iodinated iodo thyronine compounds free from carrier substances which are obtained in step a) or b), are separated from the unreacted starting material by chromatography, if X¹ or X¹ and X² of the compound of the general formula I used in step a) are bromine atoms.

9. The method according to one of claims 1 to 8, characterized in that the splitting off of the residues in step b) is carried out in a manner known per se by subjecting the reaction product of step a) to hydrolysis by the addition of a mixture of concentrated mineral acid and glacial acetic acid.

10. The method according to one of the claims 1 to 9, characterized in that the iodination of the β-ring with sodium iodide/chloramineT is carried out either before or after step b) in a manner known per se.

11. The compounds of the general formula Ia 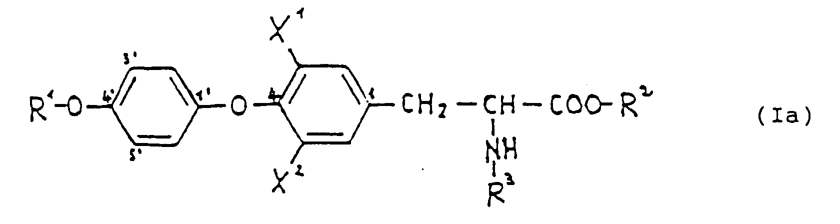 in which the
R¹ and R² are independently from each other linear or branched or cyclic alkyls with 1 to 6 carbon atoms or aryls or aralkyls with up to 8 carbon atoms,
R³ is H or an unsubstituted acyl or alkyl with up to 6 carbon atoms and
X¹ and X² stand for a Br atom and H or two bromine atoms, with the proviso that said compound is a compound other than the ethyl ester of N-acetyl-3,5-dibromo-4-p-methoxyphenoxy-D- and -L-phenylalanine.

12. The compounds according to claim 11, characterized in that R¹ and R² stand for methyl or ethyl and R³ stands for H or a formyl or acetyl.

13. The use of a compound according to one of claims 11 or 12 for introducing one or two radioactive iodine isotopes into the position 3 or the positions 3 and 5 of the α-ring of an iodo thyronine compound by a halogen exchange.

14. The use of a compound obtained in step a) or b) of claim 1 as a starting compound for preparing iodo thyronine compounds radioactively labelled in the α-ring, which are modified in the β-ring and/or in the residue Z by substitution or by another known reaction.

## Patentansprüche

1. Verfahren zur Herstellung von in der 3-Stellung bzw. den 3,5-Stellungen des α-Rings mit einem oder zwei radioaktiven Iodisotopen markierten Iodthyroninderivaten,
**gekennzeichnet durch** die Stufen:
a) Durchführung eines Halogenaustauschs bei einem 3-mono- oder 3,5-di-substituierten Thyroninderivat der allgemeinen Formel I 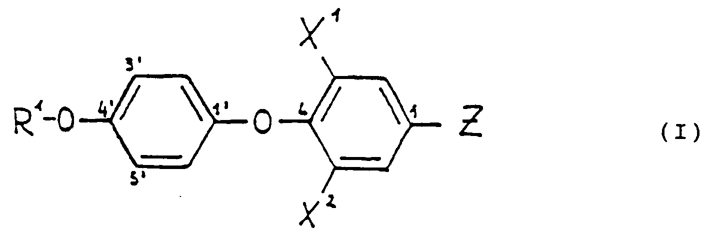 worin
R¹ für einen gegebenenfalls substituierten gerad- oder verzweigtkettigen Alkyl- oder Cycloalkylrest mit 1 bis 8 Kohlenstoffatomen, einen Phenylrest oder einen Aralkylrest mit 7 bis 14 Kohlenstoffatomen steht,
X¹ und X² gleich oder verschieden sein können und für Br, I, H, Cl, SCN, CN oder i-C₃H₇ stehen, mit der Maßgabe, daß wenigstens einer dieser Reste eine gegen ein radioaktives Iodisotop austauschbare Gruppe ist,
Z für eine der Gruppen -(CH₂)ₙ-COOR², -CH₂-CH₂-NHR³ oder -CH₂-CH(NHR³)-COOR² steht, worin
R² wie R¹ definiert ist und für den gleichen Rest wie R¹ oder einen davon verschiedenen Rest steht,
R³ für H oder einen Acylrest mit 1 bis 7 C-Atomen oder einen gerad- oder verzweigtkettigen oder cyclischen Alkylreste mit 1 bis 7 C-Atomen steht, und
n einen der Werte 0, 1 oder 2 aufweisen kann,
und wobei die Kombination der Substituenten R¹, R² und R³ so gewählt ist, daß die Verbindung einen Schmelzpunkt unterhalb von 180°C aufweist,
durch Umsetzung einer Verbindung der Formel I bei Temperaturen in der Nähe oder oberhalb ihres Schmelzpunktes mit einem geeigneten, ein radioaktives Iodisotop enthaltenden Alkali- oder Erdalkalimetalliodid, sowie gegebenenfalls Isolieren der erhaltenen Verbindung oder
b) Abspalten der Reste R¹ und/oder R² und/oder R³ aus dem Reaktionsprodukt von Stufe a) und Gewinnung der entsprechenden unsubstituierten, im α-Ring durch ein radioaktives Iodisotop markierten Iodthyroninverbindung und Isolieren des erhaltenen Reaktionsprodukts,
sowie gegebenenfalls zusätzlich
c) Einführen eines oder zweier Iodatome oder anderer Substituenten aus einer Gruppe, die aus den Resten Br, Cl, F, SCN, CN, NO₂, NH₂, OH und Alkylresten, Aralkylresten und Arylresten mit bis zu 7 Kohlenstoffatomen besteht, durch an sich bekannte Verfahren in die 3'-Stellung oder die 3',5'-Stellungen des β-Rings der in den Stufen a) oder b) erhaltenen radioiodierten Verbindungen unter Gewinnung höher substituierter, wenigstens im α-Ring mit radioaktivem Iod markierter Iodthyroninverbindungen.

2. Verfahren nach Anspruch 1, dadurch gekennzeichnet, daß in Stufe a) eine Verbindung der allgemeinen Formel I eingesetzt wird, worin Z für -CH₂-CH(NHR³)-COOR² steht und worin R¹ und R² unabhängig voneinander für Alkylreste mit 1 bis 4 Kohlenstoffatomen stehen, R³ für einen Formyl- oder Acetylrest steht und X¹ und X² unabhängig voneinander für Br- und/oder I-Atome, wobei einer dieser Reste auch für H stehen kann, stehen, und daß der Schmelzpunkt der Verbindung unter 145°C liegt.

3. Verfahren nach Anspruch 1, dadurch gekennzeichnet, daß in Stufe a) eine Verbindung der allgemeinen Formel I eingesetzt wird, in der R¹ ein Methylrest ist, R² ein Methyl- oder Ethylrest ist, R³ H oder ein Acetylrest ist und X¹ und X² beide Br oder I sind.

4. Verfahren nach Anspruch 1, dadurch gekennzeichnet, daß das in Stufe a) verwendete Alkalimetalliodid Na¹²³I, Na¹²⁵I oder Na¹³¹I ist

5. Verfahren nach Anspruch 1, dadurch gekennzeichnet, daß in Stufe a) bei Temperaturen im Bereich von 120 bis 140°C gearbeitet wird.

6. Verfahren nach Anspruch 1, dadurch gekennzeichnet, daß die Umsetzung in den Stufen a) und gegebenenfalls b) in der Spitze eines konischen Reaktionsgefäßes, einer Ampulle oder einem Schmelzpunktbestimmungsröhrchen durchgeführt wird.

7. Verfahren nach einem der Ansprüche 1 bis 6, dadurch gekennzeichnet, daß die Stufen a) und b) im gleichen Reaktionsgefäß ohne zwischenzeitliche Isolierung des Produkts der Stufe a) durchgeführt werden, indem zu dem in Stufe a) erhaltenen Reaktionsprodukt zur Abspaltung der Reste R¹, R² sowie gegebenenfalls R³ eine Säure zugesetzt und die Reaktion unter fortgesetztem Erwärmen fortgeführt wird.

8. Verfahren nach Anspruch 7, dadurch gekennzeichnet, daß dann, wenn X¹ oder X¹ und X² in der in die Stufe a) eingesetzten Verbindung der allgemeinen Formel I Bromatome sind, die in Stufe a) oder b) erhaltenen einfach oder zweifach radioiodierten trägerfreien Iodthyroninverbindungen chromatographisch von unumgesetztem Ausgangsmaterial abgetrennt werden.

9. Verfahren nach einem der Ansprüche 1 bis 8, dadurch gekennzeichnet, daß die Abspaltung der Reste in Stufe b) auf an sich bekannte Weise durch Hydrolyse des Reaktionsprodukts der Stufe a) unter Zugabe einer Mischung aus einer konzentrierten Mineralsäure mit Eisessig erfolgt.

10. Verfahren nach einem der Ansprüche 1 bis 9, dadurch gekennzeichnet, daß die Iodierung des β-Rings vor oder nach Stufe b) auf an sich bekannte Weise mit Natriumiodid/Chloramin T durchgeführt wird.

11. Verbindungen der allgemeinen Formel I 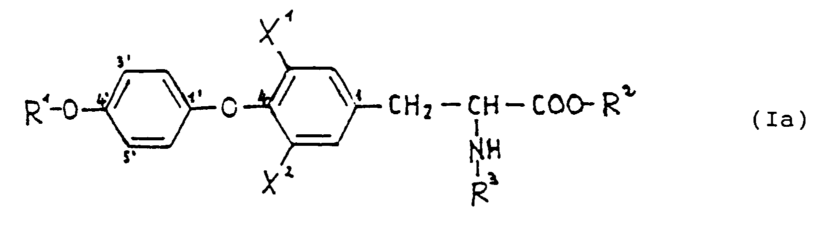 worin
R¹ und R² unabhängig voneinander für gerad- oder verzweigtkettige oder cyclische Alkylreste mit 1 bis 6 Kohlenstoffatomen oder für Aryl- oder Aralkylreste mit bis zu 8 Kohlenstoffatomen stehen,
R³ für H oder einen Acyl- oder Alkylrest mit bis zu 6 Kohlenstoffatomen steht und
X¹ und X² für ein Br-Atom und H oder zwei Bromatome stehen, mit der Maßgabe, daß die genannte Verbindung eine andere Verbindung als der Ethylester von N-Acetyl-3,5-dibrom-4-p-methoxyphenoxy-D- und -L-phenylalanin ist.

12. Verbindungen nach Anspruch 11, dadurch gekennzeichnet, daß R¹ und R² für Methyl- oder Ethylreste stehen und R³ für H oder einen Formyl- oder Acetylrest steht.

13. Verwendung einer Verbindung nach einem der Ansprüche 11 oder 12 zur Einführung eines oder zweier radioaktiver Iod-isotope in die Positionen 3 oder 3 und 5 des α-Rings einer Iodthyroninverbindung durch Halogenaustausch.

14. Verwendung einer in Stufe a) oder b) von Anspruch 1 erhaltenen Verbindung als Ausgangsverbindung für die Herstellung von im β-Ring und/oder im Rest Z durch Substitution oder durch eine andere, an sich bekannte Umsetzung modifizierten, im α-Ring radioaktiv markierten Iodthyroninverbindungen.

## Revendications

1. Méthode pour la préparation de dérivés iodo thyronine qui sont marqués en position 3 ou en positions 3,5 du cycle α avec un ou deux isotopes d'iode radioactif(s).
caractérisée par les étapes suivantes :
a) mettre en oeuvre un échange halogène dans un dérivé thyronine 3-mono- ou 3,5-di-substitué de formule générale I 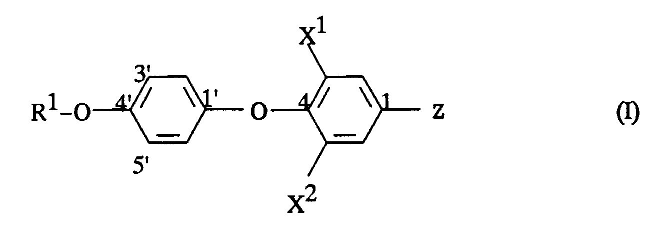 dans laquelle
R¹ est un groupe cycloalkyle ou alkyle à chaîne droite ou ramifiée ayant de 1 à 8 atomes de carbone éventuellement substituté, un groupe phényle ou un groupe aralkyle ayant de 7 à 14 atomes de carbone,
X¹ et X² peuvent être identiques ou différents et représentent les suivants : Br, I, H, Cl, SCN, CN ou i-C₃H₇, à condition qu'au moins un de ces groupes soit un groupe échangeable pour un isotope d'iode radioactif,
Z est l'un des groupes : -(CH₂)ₙ-COOR², -CH₂-CH₂-NHR³ ou -CH₂-CH(NHR³)-COOR², dans lequel
R² est défini comme R¹ et représente le même groupe que R¹ ou un groupe différent de celui-ci,
R³ représente H ou un groupe acyle ayant de 1 à 7 atomes de carbone ou un groupe alkyle à chaîne droite, ramifiée ou cyclique ayant de 1 à 7 atomes de carbone,
et
n peut être 0, 1 ou 2,
dans lequel la combinaison des substituants R¹, R² et R³ est choisie pour que le composé ait un point de fusion inférieur à 180°C, par une réaction d'un composé de formule I aux températures supérieures ou égales à leur point de fusion avec un iodure d'un métal alcalin ou d'un métal alcalino-terreux qui contient un isotope d'iode radioactif, et l'isolement éventuel du composé résultant ou
b) cliver les résidus R¹ et/ou R² et/ou R³ du produit de la réaction de l'étape (a) et récupérer le composé iodo thyronine non substitué correspondant marqué avec un isotope d'iode radioactif dans le cycle α et isoler les produits de réaction résultants et en plus éventuellement
c) introduire de manière conventionnelle 1 ou 2 atomes d'iode ou d'un autre substituant du groupe comprenant les éléments suivants : Br, Cl, F, SCN, CN, NO₂, NH₂OH et un groupe alkyle, un groupe aralkyle et un groupe aryle contenant jusqu'à 7 atomes de carbone en position 3' ou en positions 3',5' du cycle β des composés radio-iodés obtenus dans les étapes a) ou b), de façon à obtenir des composés iodo thyronine plus substitués marqués avec l'iode radioactif en au moins le cycle α.

2. Méthode selon la revendication 1, caractérisée en ce que dans l'étape a) un composé de formule générale I est utilisé, dans laquelle Z représente -CH₂-CH(NHR₃)-COOR² et dans laquelle R¹ et R² sont indépendamment l'un de l'autre un groupe alkyle ayant de 1 à 4 atomes de carbone, R³ est un groupe formyle ou acétyle et X¹ et X² sont indépendamment l'un de l'autre Br et/ou I, l'un de ces éléments pouvant être H, et en ce que le point de fusion du composé est inférieur à 145°C.

3. Méthode selon la revendication 2, caractérisée en ce que l'on utilise dans l'étape a) un composé de formule générale I, dans laquelle R¹ est un groupe méthyle, R² est un groupe méthyle ou éthyle, R³ est H ou un groupe acétyle et X¹ et X² sont simultanément Br ou I.

4. Méthode selon la revendication 1, caractérisée en ce que l'iode de métal alcalin utilisé dans l'étape a) est Na¹²³I, Na¹²⁵I ou Na¹³¹I.

5. Méthode selon la revendication 1 caractérisée en ce que l'étape a) est mise en oeuvre à une température de 120° à 140°C.

6. Méthode selon la revendication 1, caractérisée en ce que la réaction de l'étape a) et éventuellement l'étape b) est mise en oeuvre dans le col d'un erlenmeyer, dans une ampoule ou dans un tube à point de fusion.

7. Méthode selon l'une des revendications 1 à 6, caractérisée en ce que les étapes a) et b) sont mises en oeuvre dans le même récipient de réaction sans isoler d'intermédiaire du produit de l'étape a), dans laquelle pour cliver les éléments R¹, R² et éventuellement R³ on ajoute un acide au produit de réaction obtenu dans l'étape a) et la réaction est poursuivie pendant que le chauffage est maintenu.

8. Méthode selon la revendication 7, caractérisée en ce que les composés iodo thyronine mono- ou di-radio iodé libre de substances porteuses qui sont obtenues dans l'étape a) ou b), sont séparés du produit de départ n'ayant pas réagi par une chromatographie, si X¹ ou X¹ et X² dans le composé de formule générale I utilisé dans l'étape a) sont des atomes de brome.

9. Méthode selon l'une des revendications 1 à 8, caractérisée en ce que le clivage des résidus dans l'étape b) est mise en oeuvre d'une manière connue en soi en soumettant le produit de réaction de l'étape a) à une hydrolyse par l'addition d'un mélange d'un acide minéral concentré et d'acide acétique glacial.

10. Méthode selon l'une des revendications 1 à 9, caractérisée en ce que l'iodination du cycle β avec de l'iode de sodium/chloramine T est mise en oeuvre soit avant soit après l'étape b) d'une manière connue en soi.

11. Composés de formule générale Ia : 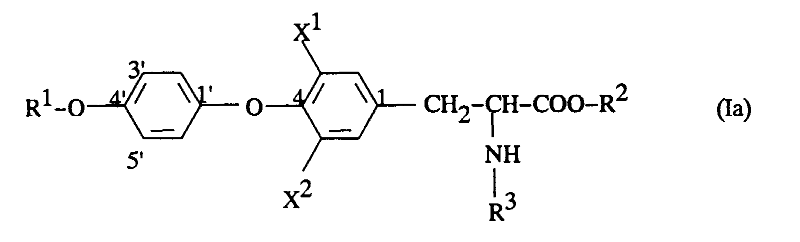 dans laquelle
R¹ et R² sont indépendamment l'un de l'autre un groupe alkyle à chaîne droite, ramifiée, ou cyclique ayant de 1 à 6 atomes de carbone ou un groupe aryle ou aralkyle ayant jusqu'à 8 atomes de carbone,
R³ est H ou un groupe alkyle ou acyle non substitué ayant jusqu'à 6 atomes de carbone et
X¹ et X² représentent un atome de brome et H ou deux atomes de brome, à condition que ledit composé soit un composé autre que l'ester éthylique de N-acétyl-3,5-dibromo-4-p-méthoxyphénoxy-D-phénylalanine et de N-acétyl-3,5-dibromo-4-p-méthoxyphénoxy-L-phénylalanine.

12. Composés selon la revendication 11, caractérisés en ce que R¹ et R² représentent un groupe méthyle ou éthyle et R³ représente H ou un groupe formyle ou acétyle.

13. Utilisation d'un composé selon l'une des revendications 11 ou 12 pour l'introduction d'un ou deux isotopes iode radioactifs en position 3 ou en positions 3 et 5 d'un cycle α d'un composé iodo thyronine par un échange d'halogène.

14. Utilisation d'un composé obtenu à l'étape a) ou b) de la revendication 1 comme composé de départ pour la préparation de composés iodo thyronine radioactivement marqués dans le cycle α, qui sont modifiés dans le cycle β et/ou dans le groupe Z par une substitution ou par une autre réaction connue.
